(19) Europäisches Patentamt
European Patent Office
Office européen des brevets

(11) Numéro de publication: **0 421 861 A1**

(12) **DEMANDE DE BREVET EUROPEEN**

(21) Numéro de dépôt: **90402711.7**

(22) Date de dépôt: **01.10.90**

(51) Int. Cl.5: **C07D 491/04**, C07D 495/04, A61K 31/40, A61K 31/435, A61K 31/55, //(C07D491/04, 307:00,209:00),(C07D491/04, 307:00,221:00),(C07D491/04, 307:00,223:00),(C07D495/04, 333:00,209:00),(C07D495/04, 333:00,221:00),(C07D495/04, 333:00,223:00)

(30) Priorité: **02.10.89 FR 8912854**

(43) Date de publication de la demande: **10.04.91 Bulletin 91/15**

(84) Etats contractants désignés: **AT BE CH DE DK ES FR GB GR IT LI LU NL SE**

(71) Demandeur: **SANOFI**
**40, Avenue George V**
**F-75008 Paris(FR)**

(72) Inventeur: **Badorc, Alain**
**1 rue La Canal**
**F-31120 Roquettes(FR)**

Inventeur: **Bordes, Marie-Françoise**
**190 Avenue du Comminges**
**F-31860 Labarthe-sur-Seze(FR)**

Inventeur: **Frehel, Daniel**
**100 Allée de Barcelone, Appart. 206**
**Résidence l'Autan, F-31000 Toulouse(FR)**

Inventeur: **Herbert, Jean-Marc**
**4 rue du Cantou Caout**
**F-31830 Plaisance du Touch(FR)**

(74) Mandataire: **Varady, Peter et al**
**Cabinet Lavoix 2, Place d'Estienne d'Orves**
**F-75441 Paris Cedex 09(FR)**

(54) **Dérivés d'hydroxy-2 thiophène et furanne condensés avec un cycle azoté, leur procédé de préparation et leur application en thérapeutique.**

(57) L'invention a pour objet des composés de formule

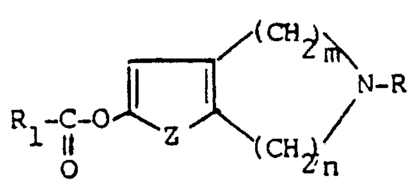

I

dans laquelle $R_1$ est choisi parmi les groupes $R_3$ et $OR_3$, $R_3$ étant un alkyle, phényle ou benzyle, R est choisi parmi H et un groupe $CHR_2R'$ dans lequel $R_2$ est choisi parmi H, les groupes alkyle, carboxylique ou carboxamido et R′ est H, un groupe alkyle ou phényle, Z représente S ou O; m et n, identiques ou différents, valent 1 ou 2, ainsi que leurs N-oxydes, leurs sels et ammonium quaternaires.

L'invention a également pour objet le procédé de préparation des composés de formule I ainsi que des compositions pharmaceutiques les contenant.

EP 0 421 861 A1

La présente invention concerne des dérivés d'hydroxy-2 thiophène et furanne condensés avec une amine hétérocyclique de formule :

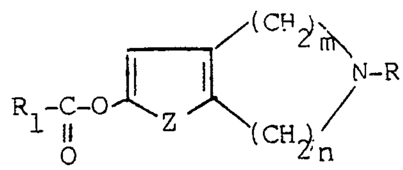

I

dans laquelle $R_1$ est choisi parmi les groupes $R_3$ et $OR_3$ dans lesquels $R_3$ est choisi parmi les groupes alkyle en $C_1$ à $C_6$, phényle et benzyle;
R est choisi parmi H et les groupes $CHR_2R'$ dans lesquels $R_2$ est choisi parmi H, les groupes alkyle en $C_1$ à $C_4$, les groupes $-COOR_4$, $R_4$ étant choisi parmi H, les groupes alkyle en $C_1$ à $C_4$ et benzyle, les groupes $-CONR_5R_6$, $R_5$ et $R_6$ indépendamment l'un de l'autre étant choisis parmi H, les groupes alkyle en $C_1$ à $C_4$ et benzyle ou $R_5$ et $R_6$ forment avec l'azote auquel ils sont rattachés un hétérocyle saturé en $C_4$-$C_8$, notamment pipéridino, pyrrolidinyl-1, morpholino et pipérazinyl-1; $R'$ est choisi parmi H, les groupes alkyle en $C_1$ à $C_4$ et les groupes phényle substitué ou non substitué;
Z représente S ou O; m et n, identiques ou différents, valent 1 ou 2,
ainsi que les N-oxydes de ces dérivés, les sels des amines et de leurs oxydes avec les acides pharmaceutiquement acceptables, et les ammoniums quaternaires, formés avec des halogénures et sulfates d'alkyles en $C_1$ à $C_4$ et ces amines.

Les groupes phényles peuvent être substitués par les atomes d'halogène, les groupes alkyle ou alkoxy en $C_1$ à $C_4$, le groupe trifluorométhyle ou le groupe nitro.

L'invention concerne aussi les procédés de préparation de ces composés et les compositions pharmaceutiques comprenant ces composés. Les composés de formule I dans laquelle $R_1$ représente un groupe alkyle en $C_2$ à $C_4$ sont préférés et particulièrement ceux pour lesquels Z est S, $R_2$ est H et $R'$ est un groupe phényle substitué ou non.

Les composés, selon l'invention, de formule I, peuvent être préparés par O-acylation des composés de formule II

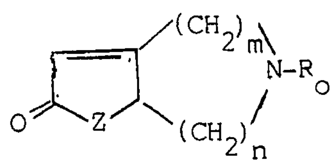

II

dans laquelle Z, m, n, ont la même signification que dans la formule I et $R_o$ représente un groupe protecteur de la fonction amine, tel que la liaison N-$R_o$ soit stable en présence d'une base forte et $R_o$ éliminable en milieu acide, comme par exemple les groupes trityle ou tertiobutyloxycarbonyle,
l'acylation étant suivie de l'élimination du groupe $R_o$ par action d'un acide anhydre, de façon à obtenir un composé de formule III

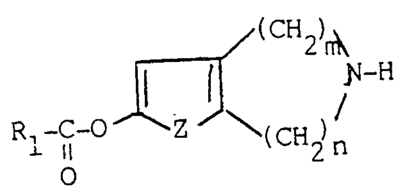

III

et lorsque R est différent de H, on fait ensuite réagir, sur le composé de formule III un composé de formule

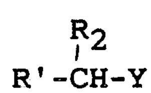

dans laquelle Y représente un halogène ou un groupe $R_7SO_3$, $R_7$ étant choisi parmi les groupes alkyle en $C_1$ à $C_4$ et phényle.

L'acylation peut être notamment effectuée par action d'un dérivé réactif de l'acide $R_1COOH$, tel qu'un halogénure d'acide, un anhydride de l'acide ou un carbonate ou un chloroformiate convenable sur l'énolate de formule IV

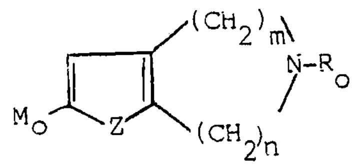 IV

dans laquelle M représente un cation alcalin,
qui est obtenu par action d'une base forte, telle que le butyllithium, un tertiobutylate alcalin ou un hydrure alcalin, sur le composé de formule II; la réaction est effectuée dans un solvant inerte, tel qu'un éther, par exemple le tétrahydrofuranne, un hydrocarbure aliphatique, par exemple l'hexane ou le pentane, ou leurs mélanges, à une température comprise entre -5° C et 30° C environ et de préférence en atmosphère inerte.

Dans le cas où le groupe $R_o$ est un groupe protecteur, son élimination est effectuée par action à température ambiante de l'acide trifluoroacétique pur ou dans un solvant tel que le dichlorométhane, ou par action de l'acide acétique à chaud ou encore par action d'un acide halogènhydrique, en solution dans un solvant organique, tel que HCl 3N dans $CH_3COOC_2H_5$.

L'alkylation de l'azote du composé de formule III est effectuée dans les conditions habituelles par action d'un halogénure ou d'un sulfonate convenable de formule $YCHR_2R'$ en présence d'une base telle que $NaHCO_3$ ou $KHCO_3$, dans un solvant aprotique polaire, comme le tétrahydrofuranne ou le diméthylformamide, de préférence en atmosphère inerte et à une température comprise entre 20° C et 90° C.

Dans les cas où $R_2$ représente H ou un groupe alkyle en $C_1$ à $C_4$, on peut également effectuer l'acylation d'un composé de formule II dans laquelle $R_O$ représente alors $-CHR_2R'$, $R'$ étant tel que défini précédemment pour obtenir directement un composé de formule I.

Lorsque $R_1$ représente $CH_3$, l'acylation du composé de formule II peut aussi être effectuée par action de l'acétate d'isopropényle

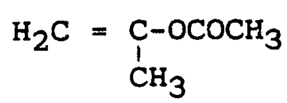

en excès, sur le composé de formule II en présence d'un catalyseur acide, éventuellement dans un solvant tel qu'un éther comme le tétrahydrofuranne ou un hydrocarbure comme le benzène ou l'éther de pétrole; les catalyseurs acides sont notamment les acides benzènesulfonique ou para-toluènesulfonique; la réaction est effectuée de préférence à la température de reflux du solvant.

Les N-oxydes des composés de formule I sont préparés par action d'un peroxyacide organique sur l'amine de formule I dans les conditions habituelles. On peut notamment effectuer l'oxydation par action d'au moins un équivalent molaire des peracides commerciaux : l'acide peracétique, l'acide perbenzoïque ou l'acide chloro-3 perbenzoïque, sur l'amine de formule I en solution dans un solvant inerte, tel que le dichlorométhane ou le chloroforme, à une température comprise entre -5° C et 30° C; on peut aussi mettre en oeuvre des peracides préparés extemporanément en appliquant des méthodes connues.

Les N-oxydes sont peu stables et on les isole de préférence sous la forme d'un de leurs sels d'addition avec un acide, notamment un acide halogènhydrique, HCl ou HBr; pour cela, on introduit dans le milieu d'oxydation en fin de réaction, l'acide avant de précipiter le sel par addition dans le milieu d'un solvant dans lequel il n'est pas soluble, tel que l'éther éthylique.

Les sels d'ammonium quaternaire sont préparés de façon classique par action d'un halogénure ou d'un sulfate d'alkyle sur l'amine de formule I en solution.

Enfin les sels d'amines des composés de formule I sont obtenus par l'action d'au moins un équivalent de l'acide pharmaceutiquement aoceptable sur l'amine de formule I en solution, et isolés de façon classique par précipitation ou élimination des solvants.

Certains des composés de formule II sont connus; les autres peuvent être préparés en appliquant des méthodes déjà décrites pour des composés analogues.

Ainsi le composé de formule II dans laquelle Z = S, m = 1, n = 2 et $R_o$ = $C(C_6H_5)_3$ est décrit dans le

brevet FR-A-2 576 901; des composés pour lesquels Z = S, m = 1, n = 2 et $R_o$ = $CH_2R'$ sont décrits dans FR-A-2 508 459 tandis que des composés dans lesquels Z = S, m = 1, n = 2 et $R_o$ = $R'$-CH-COOR sont décrits dans FR-A-2 576 901.

Les composés pour lesquels Z = S, m = 2, n = 1 sont préparés en appliquant les mêmes méthodes.

Le composé de formule II pour laquelle Z = 0, m = 2, n = 1 et $R_o$ = $COOCH_2C_6H_5$ est décrit dans Chem. Pharm. Bull. 30(3) p. 1084-87 (1982) et le principe de sa méthode de préparation peut être appliqué pour préparer, par exemple, les composés dans lesquels $R_o$ = $C(C_6H_5)_3$, $COO(tC_4H_9)$, $CH_2R'$.

Les composés de formule II dans laquelle Z = 0, m = 1, n = 2 et $R_o$ représente $CH_2R'$ ou un groupe protecteur peuvent être préparés par aminoalcoylation de l'acide glyoxylique, en appliquant une méthode décrite par J. Schreiber et Coll. dans Bull. Soc. Chim. France (1963) p. 625-629, selon le schéma réactionnel suivant :

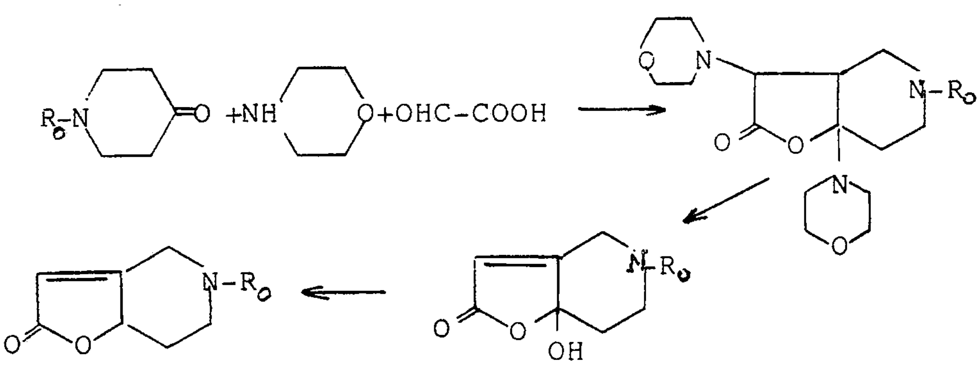

Les composés de formule II dans laquelle Z = S, m = 1, n = 1 et $R_o$ représente $CH_2R'$ ou un groupe protecteur peuvent être préparés à partir de l'aldéhyde thénoïque en appliquant le schéma réactionnel suivant :

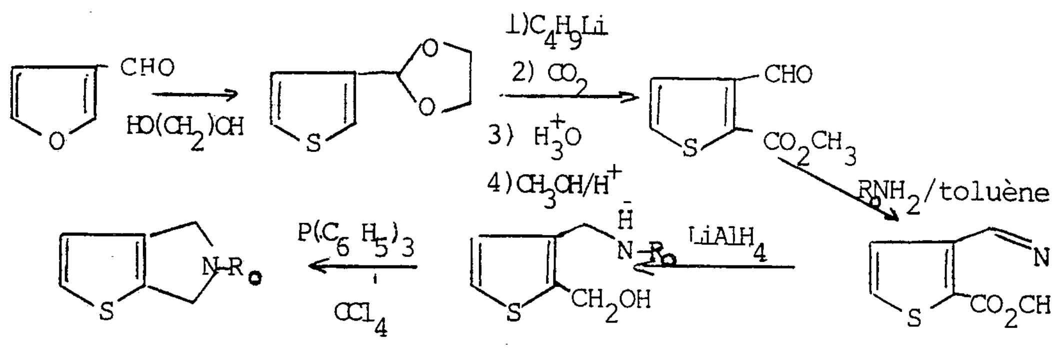


On connaît des composés de formule II dans laquelle $R_o$ représente $R'$-CH-$R_2$, Z = S, m = 1, n = 2 et $R'$ représente le groupe phényle, qui sont des inhibiteurs de l'agrégation des plaquettes sanguines et des agents antithrombotiques : les composés dans lesquels $R_2$ représente $COOR_4$ ou $-CONR_5R_6$, sont revendiqués dans le brevet FR-A-2 576 901, tandis que les composés dans lesquels $R_2$ représente H, sont revendiqués dans le brevet FR-A-2 495 156.

Les composés de l'invention quant à eux présentent une activité inhibitrice des élastases pancréatique et leucocytaire, enzymes protéolytiques pouvant hydrolyser la plupart des composants des tissus conjonctifs et tout particulièrement l'élastine.

L'implication de l'élastase leucocytaire dans la pathogénie de certaines maladies et en conséquence l'intérêt de ses inhibiteurs a été souligné; on peut se reporter notament à la courte revue parue dans Annual Reports in Medicinal Chemistry vol. 20, p. 237 à 246 (1985).

Ainsi, on sait que l'administration d'inhibiteurs de l'élastase diminue la destruction du tissu pulmonaire dans l'emphysème pulmonaire comme dans la fibrose kystique.

On a aussi montré que les modifications des vaisseaux sanguins avec l'âge étaient en partie liées à l'augmentation de la synthèse d'une élastase dans leur paroi, entraînant la dégradation du tissu conjonctif de la paroi vasculaire et favorisant la formation des plaques d'athérome. L'administration d'un inhibiteur

d'élastase sera donc utile dans la prévention et le traitement de l'athérosclérose.

On a enfin récemment mis en évidence un déséquilibre entre les protéases ayant une activité élastolytique et leurs inhibiteurs naturels dans les troubles rhumatismaux dégénératifs et certains processus inflammatoires. Ainsi, l'administration d'un inhibiteur de l'élastase, en rétablissant l'équilibre biologique de ces enzymes sera utile dans le traitement de ces maladies.

D'autre part, il est connu que l'administration d'un inhibiteur de l'élastase pancréatique est utile dans le traitement des pancréatites aïgues.

Par ailleurs, certains des composés de l'invention, et notamment les composés de formule I dans laquelle Z = S, m = 1, n = 2 et $R_2$ = H ou $COOR_4$ avec $R_4$ = alkyle en $C_1$-$C_4$ et $R'$ = aryle ont en outre une activité antithrombotique, associée parfois à une activité antiagrégante plaquettaire.

Etant donné les activités pharmacologiques des composés selon l'invention, les compositions pharmaceutiques qui les comprennent seront notamment utiles dans le traitement de l'emphysème pulmonaire, de l'athérosclérose, de la mucoviscidose, des états inflammatoires articulaires et dans tous les états où il existe un déséquilibre pathologique entre les élastases et leurs inhibiteurs naturels.

Les compositions pharmaceutiques comprenant au moins un composé de formule I, son N-oxyde, leurs sels avec un acide minéral ou organique pharmaceutiquement acceptable ou ses ammoniums quaternaires, associé aux excipients ou véhicules usuels sont par conséquent un autre objet de l'invention. Ces compositions peuvent être administrées selon les voies habituelles, notamment par voie orale, par exemple sous forme de comprimés, de gélules, de granules ou de solutions, ou par voie injectable; les formes pharmaceutiques seront préparées par des méthodes bien connues du spécialiste, avec les excipients usuels compatibles. Les doses administrables peuvent se situer notamment entre 10 et 250 mg de principe actif par voie orale par jour.

Dans ce qui suit, on décrit des exemples de composés de l'invention et de leurs procédés de préparation et on indique les résultats de leur étude pharmacologique.

Les analyses centésimales et les spectres infra-rouge et RMN (étalon interne tétraméthylsilane) ont confirmé la structure des produits isolés.

EXEMPLE 1 :

Composé de formule I dans laquelle Z = S; m = 1; n = 2; $R_2$ = H; $R'$ = 2 -$ClC_6H_4$; $R_1$ = $CH_3$ - (référence : PCR 3927).

a) (chloro-2 benzyl)-5 tétrahydro-5,6,7,7a 4H-thiéno[3,2-c] pyridine-one-2 (formule II : Z = S; m = 1; n = 2; R = $CH_2(2\text{-}ClC_6H_4)$).

Dans une solution refroidie à -20° C de 32,6g (0,123 mole) de (chloro-2 benzyl)-5 tétrahydro-4,5,6,7 thiéno[3,2,c] pyridine dans 320 $cm^3$ de tétrahydrofuranne on introduit, goutte à goutte, 79 $cm^3$ d'une solution de butyllithium à 12% dans l'hexane (0,147 mole). A la fin de l'addition le dérivé lithié précipite et on laisse revenir la température à 0° C. On ajoute alors 15 $cm^3$ d'hexaméthylphosphotriamide (HMPT).; le précipité devient rouge foncé.

La température est abaissée à -40° C et on ajoute, goutte à goutte, en une demi-heure, une solution de borate de tributyle (39,8 $cm^3$ : 0,147 mole) dans 40 $cm^3$ de tétrahydrofuranne anhydre. Le précipité disparaît et le milieu réactionnel devient jaune clair. La température est maintenue à -40° C pendant une demi-heure puis ramenée à 10° C et maintenue pendant 2 heures à cette température. On ajoute, goutte à goutte, 33 $cm^3$ (0,291 mole) d'eau oxygénée à 30% en maintenant la température du milieu inférieure à 30° C. Un précipité abondant se forme en cours d'addition. L'agitation est maintenue pendant 1 heure à température ambiante. On verse le milieu réactionnel sur de l'eau, extrait par 3 x 200 $cm^3$ d'éther éthylique, sèche les phases organiques sur sulfate de sodium et concentre sous vide à une température infé-rieure à 40° C. Le liquide résiduel est chromatographié sur colonne de silice (éluant : cyclohexane-acétate d'éthyle 6/4) pour éliminer le HMPT restant.

Après évaporation du solvent, on traite par un équivalent molaire d'acide oxalique dans l'acétone, et filtre les cristaux jaune clair formés.

Par recristallisation dans l'éthanol, on obtient des cristaux d'oxalate de couleur beige.

Rendement : 52 %, F : 170° C. Base : F : 73° - 74,5° C (éthanol)

RMN ($CDCl_3$) : 7,1-7,6(m,4H); 6,2(s,1H); 4,2-4,7(m,1H); 3,9(s,2H); 1,5-4,2(m,6H).

b) acétate de (chloro-2 benzyl)-5 tétrahydro-5,6,7,7a 4H-thiéno[3,2-c] pyridyle-2.

On dissout 8,85 g (0,0316 mole) de (chloro-2 benzyl)-5 tétrahydro-5,6,7,7a 4H-thiéno[3,2-c] pyridine-one-2 dans 120 ml d'acétate d'isopropényle avec 7,8g (0,0411 mole) d'acide p-toluènesulfonique; le milieu est maintenu à 90 °C pendant 6 heures, sous agitation. Après refroidissement vers 20 °C, on introduit 2 volumes d'eau dans le milieu et alcalinise par addition d'une solution aqueuse saturée de $NaHCO_3$ avant d'extraire le produit recherché avec l'acétate d'éthyle.

Après élimination du solvant, l'huile dissoute dans $CH_2Cl_2$ est filtrée sur silice pour donner, avec 68 % de rendement l'ester qui fond à 68 °C.

RMN$^1$H : (60MHz, $CDCl_3$) : 2,20(s,3H); 2,63-2,95(m,4H); 3,51(s,2H); 3,80(s,2H); 6,27(s,1H); 7,07-7,67(m,1H).

EXEMPLE 2

Composé de formule I dans laquelle Z = S; m = 1; n = 2; $R_2$ = H; R' = 2-$ClC_6H_4$; $R_1$ = $(CH_3)_3C$ (référence : SR 26766).

A 0 °C, on ajoute, goutte à goutte, 10,7 ml (0,017 mole) d'une solution 1,6M de butyllithium dans l'hexane, dans une solution de 4,55 g (0,0162 mole) de (chloro-2 benzyl)-5 tétrahydro-5,6,7,7a 4H-thiéno-[3,2-c] pyridine-one-2 dans 45 ml de tétrahydrofuranne.

Après 15 minutes d'agitation toujours à 0 °C, on introduit goutte à goutte 2,04 ml (0,0166 mole) de chlorure de pivaloyle puis laisse le milieu sous agitation pendant 1 heure 30 à la température ambiante, soit environ à 20 °C. On introduit alors 2 volumes d'eau et on extrait le produit final dans l'acétate d'éthyle; l'huile isolée après lavage à l'eau de la phase organique et évaporation du solvant est chromatographiée sur une colonne de silice, en éluant avec un mélange toluène/acétate d'éthyle (9/1-v/v). Le chlorhydrate du produit final, isolé avec 57% de rendement, fond à 214 °C.

On peut aussi préparer l'énolate de la thiénopyridine-one-2 comme suit : on introduit lentement à 5 °C, 5g (0,0179 mole) de la thiénopyridine-one-2 en solution dans 50 ml de tétrahydrofuranne dans 20 ml de ce même solvant contenant 2,11g (0,0188 mole) de tertiobutylate de potassium. Après quelques minutes d'agitation, 2,3 ml (0,0188 mole) de chlorure de pivaloyle sont ajoutés. Après 2 heures d'agitation à température ambiante, on introduit dans le milieu 2 volumes d'eau, puis 2 volumes d'acétate d'éthyle. Après agitation, la phase organique est décantée puis séparée, lavée avec de l'eau, séchée, le solvant éliminé et on isole le produit final pur comme précédemment.

EXEMPLES 3 à 12 :

On prépare, en appliquant l'une des méthodes décrites à l'exemple 2 en mettant en oeuvre le chlorure d'acide $R_1COCl$, $R_1$ étant tel que défini précedemment, les composés de formule I ayant Z = S; m = 1; n = 2, dont les caractéristiques sont les suivantes :

| Ex. | SR | $R_1$ | $R_2$ | R' | F (chlorhydrate) | rendement en sel |
|---|---|---|---|---|---|---|
| 3 | PCR4094 | $CH_3(CH_2)_2$ | H | 2-$ClC_6H_4$ | 181 °C | 65 % |
| 4 | 26772 | $(CH_3)_2CH$ | H | 2-$ClC_6H_4$ | 189 °C | 60 % |
| 5 | 26851 | $(CH_3)_2CH(CH_2)$ | H | 2-$ClC_6H_4$ | 190 °C | 45 % |
| 6 | 26769 | $(CH_3)_2CH$ | H | 4-$ClC_6H_4$ | 220 °C | 48 % |
| 7 | 26768 | $(CH_3)_3C$ | H | 4-$ClC_6H_4$ | 248 °C | 61 % |
| 8 | 26767 | $CH_3(CH_2)_2$ | H | 4-$ClC_6H_4$ | 225 °C | 65 % |
| 9 | 26829 | $(CH_3)_2CH$ | H | $C_6H_5$ | 220 °C | 30 % |
| 10 | 26827 | $(CH_3)_3C$ | H | $C_6H_5$ | 234 °C | 40 % |
| 11 | 27261 | $(CH_3)_3C$ | H | 4-$OCH_3C_6H_4$ | 255 °C | 85 % |
| 12 | 26915 | $C_6H_5$ | H | 2-$ClC_6H_4$ | 202 °C(oxal.) | 49 % |

Les déplacements chimiques pour les protons des composés des exemples 3 à 12 sont donnés dans ce qui suit :

| Ex. (sel) | RMN 1H - δ(ppm) : |
|---|---|
| 3 (HCl) | (80 MHz,DMSOd6) : 1,05(t,3H) ; 1,44-2,01(m,2H) ; 3,04-3,95(m,4H) ; 4,30(s,2H) ; 4,70(s,2H) ; 6.73(s,1H) ; 7,43-7,84(m,3H) ; 8,06-8,34(m,1H) |
| 4 (HCl) | (80 MHz,DMSOd6) : 1,29(d,6H) ; 3,04(h,1H) ; 3,15-3,68(m,4H) ; 4,28(s,2H) ; 4,69(s,2H) ; 6,72(s,1H) ; 7,51-7,65(m,3H) ; 8,14-8,24(m,1H) |
| 5 (HCl) | (250 MHz,DMSOd6) : 0,94(d,6H) ; 2,07(m,1H) ; 2,49(d,2H) ; 3,03-3,66(m,4H) ; 4,18(s,2H) ; 4,59(s,2H) ; 6,61(s,1H) ; 7,46-7,60(m,3H) ; 8,06-8,34(m,1H) |
| 6 (HCl) | (80 MHz,DMSOd6) : 1,31(d,6H) ; 2,97(h,1H) ; 3,16-3,82(m,4H) ; 4,19(s,2H) ; 4,57(s,2H) ; 7,64(d,2H) ; 7,82(d,2H) |
| 7 (HCl) | (80 MHz,DMSOd6) : 1,36(s,9H) ; 2,97-3,67(m,4H) ; 4,17(s,2H) ; 4,57(s,2H) ; 6,69(s,1H) ; 7,73(d,2H) ; 7,84(d,2H) |
| 8 (HCl) | (80 MHz,DMSOd6) : 1,03(t,3H) ; 1,72(m,2H) ; 2,66(t,2H) ; 4,13(s,2H) ; 4,54(s,2H) ; 6,68(s,1H) ; 7,64(d,2H) ; 7,84(d,2H) |
| 9 (HCl) | (250 MHz,DMSOd6) : 1,19(d,6H) ; 2,85(h,1H) ; 3,04-3,12(m,2H) ; 3,51-3,66(m,2H) ; 4,07(s,2H) ; 4,41-4,47(m,2H) ; 6,60(s,1H) ; 7,46-7,48(m,3H) ; 7,64-7,66(m,2H) |
| 10 (HCl) | (250 MHz,DMSOd6) : 1,26(s,9H) ; 3,03-3,66(m,4H) ; 4,09(s,2H) ; 4,45(s,2H) ; 6,61(s,1H) ; 7,47-7,49(m,3H) ; 7,62-7,64(m,2H) |
| 11 (HCl) | (250 MHz,DMSOd6) : 1,27(s,9H) ; 2,96-3,19(m,2H) ; 3,52-3,74(m,2H) ; 3,81(s,3H) ; 4,03(s,2H) ; 4,38(s,2H) ; 6,61(s,1H) ; 7,06(d,2H) ; 7,58(d,2H) |
| 12 (amine) | (80 MHz,DMSOd6) 2,74-3,21(m,4H) ; 3,75(s,2H) ; 4,05(s,2H) ; 6,77(s,1H) ; 7,33-7,93(m,7H) ; 8,06-8,34(m,2H) |

Les thiénopyridine-one-2 intermédiaires ont été préparées en appliquant la méthode décrite dans l'exemple 1 a) et transformées en sels :

- le maléate de la (chloro-4 benzyl)-5 tétrahydro-5,6,7,7a 4H-thiéno[3,2-c]pyridine-one-2 fond à 158° C,
- le maléate de la benzyl-5 tétrahydro-5,6,7,7a 4H-thiéno[3,2-c] pyridine-one-2 fond à 132° C,
- l'oxalate de la (méthoxy-4 benzyl)-5 tétrahydro-5,6,7,7a 4H-thiéno[3,2-c] pyridine-one-2 fond à 125° C.

EXEMPLE 13 :

Composé de formule I dans laquelle Z = S; m = 1; n = 2; $R_2$ = $CH_3$; R′ = $C_6H_5$; $R_1$ = $C(CH_3)_3$ - (référence : SR 27319).

a) (phényl-1 éthyl)-5 tétrahydro-5,6,7,7a 4H- thiéno[3,2-c] pyridine-one-2 (composé de formule II dans laquelle Z = S; m = 1; n = 2; R = $CH(CH_3)C_6H_5$).

A une solution de 10g (0,052 mole) de chlorhydrate de tétrahydro-5,6,7,7a 4H-thiéno[3,2-c] pyridine-one-2 dans 100 ml de diméthylformamide, on ajoute 10,43g (0,104 mole) de bicarbonate de potassium et, 7,82 g (0,052 mole) d'iodure de sodium puis 9,8 g (0,053 mole) de bromo-1 éthyl benzène. Le milieu est porté à 60° C pendant 90 minutes puis versé dans 600 ml d'eau. Le produit final est extrait dans l'acétate d'éthyle. Rendement 66%.

b) On prépare l'ester de formule I par l'intermédiaire du dérivé lithié de la thiénopyridine-one, en appliquant le mode opératoire décrit à l'exemple 2. Le chlorhydrate, mélange de stéréoisomères, fond à 133° C.

RMN$^1$H (250 MHz, DMSOd6) : 1,25(d,9H); 1,74-1,80(m,3H); 2,82-2,95(m,1H); 2,95-3,10(m,1H); 3,25-3,45-(m,2H); 3,90-4,05(m,1H); 4,05-4,20(m,1H); 4,40-4,55(m,1H); 4,57-4,70(m,1H); 6,55(d,1H); 7,45(s,3H); 7,66-7,75(m,2H); 11,80-12,00(d,1H).

EXEMPLES 14 et 15

On prépare, en appliquant le mode opératoire décrit à l'exemple 13, les composés de formule I dans laquelle Z = S; m = 1; n = 2; R′ = H; $R_1$ = $-C(CH_3)_3$ et $R_2$ = H ou $R_2$ = $CH_2CH_3$, dont les caractéristiques sont les suivantes :

a) ex. 14 (SR 26984) :

t-butyrate de méthyl-5 tétrahydro-4,5,6,7 thiéno[3,2-c] pyridyle-2 : le sel d'acide oxalique de ce composé obtenu par action d'1 molécule d'acide oxalique pour 1 molécule d'amine de formule I fond à 170° C.

RMN$^1$H (250 MHz,DMSOd6) : 1,27(s,9H); 4,80(s,3H); 2,97(t,2H); 3,36(t,2H); 4,05(s,2H); 6,60(s,1H).

L'oxalate de la thiénopyridine-one-2 de départ fondait à 145° C.

b) ex. 15 (SR 27318) :

t-butyrate de (propyl-5) tétrahydro-4,5,6,7 thiéno[3,2-c] pyridyle-2 : le chlorhydrate de ce composé fond à 190° C.

RMN$^1$H (250 MHz, DMSOd6) : 0,95(t,3H); 1,30(s,9H); 1,72-1,87(m,2H); 3,05-3,17(m,5H); 3,60-3,70 (m,1H); 4,00-4,05(m,1H); 4,30(d,1H); 6,58(s,1H); 11,35(s,1H).

EXEMPLE 16

Composé de formule I dans laquelle Z = S; m = 1; n =2; R = H; $R_1$ = $-C(CH_3)_3$ (référence SR 26842).

a) t-butyrate de (trityl-5) tétrahydro-4,5,6,7 thiéno[3,2-c] pyridyle-2 :

Préparé en appliquant le mode opératoire décrit à l'exemple 2 à partir de la (trityl-5) tétrahydro-5,6,7,7a 4H-thiéno[3,2-c] pyridine-one-2 (f = 210° C préparé comme dans FR-A-2 576 901), de butyllithium et de chlorure de pivaloyle.

B) t-butyrate de tetrahydro-4,5,6,7 thieno[3,2-C] pyridyle-2 :

On dissout 1,3g de derive obtenu selon a) dans 10 ml de chlorure de méthylène et on ajoute, goutte à goutte, 1 ml d'acide trifluoroacétique; après 30 minutes d'agitation, le mélange est versé dans au moins 3 équivalents d'une solution 5,8N de HCl=I dans l'éther éthylique; le précipité formé est isolé.

On obtient ainsi avec 74% de rendement, le chlorhydrate de ce composé qui fond à 211° C.

RMN$^1$H (250 MHz,DMSOd6) : 1,27(s,9H); 2,92-2,96(m,2H); 3,33-3,39(m,2H); 4,05(s,2H); 6,61(s,1H).

EXEMPLE 17

Composé de formule I dans laquelle Z = S; m = 1; n = 2; R = H; $R_1$ = $(CH_3)_2CH$ (référence SR 26861).

Le chlorhydrate de l'isobutyrate de tétrahydro-4,5,6,7 thiéno[3,2-c] pyridyle-2, obtenu en appliquant le mode opératoire décrit à l' exemple 16 en remplaçant le chlorure de pivaloyle par le chlorure d'isobutyryle, fond à 181° C.

EXEMPLE 18

Composé de formule I dans laquelle Z = 0; m = 1; n = 2; $R_2$ = H; $R'$ = 2-$ClC_6H_4$; $R_1$ = $-C(CH_3)_3$ - (référence SR 26938).

a) (chloro-2 benzyl)-5 dimorpholino-3,7a hexahydro-3a,4,5,6,7,7a 3H-furo[3,2-c] pyridine-one-2 :

A une solution de 16,46 g (0,179 mole) d'acide glyoxylique monohydraté dans 40 cm$^3$ d'éthanol à 95%, refroidie à 0° C, on ajoute goutte à goutte 31,16g (0,358 mole) de morpholine. On laisse revenir à

température ambiante et on ajoute rapidement, par portions, 40 g (0,179 mole) de N-(chloro-2 benzyl) pipéridine-one-4. On observe une dissolution partielle puis un précipité se forme dans le milieu réactionnel.

On agite pendant 3 heures à température ambiante et on ajoute 100 ml d'eau, filtre le précipitéformé, et le lave à l'eau puis à l'isopropanol.

Après séchage, on recueille 31,8 g de poudre blanche de F = 173° C (rendement : 41%).

b) (chloro-2 benzyl)-5 hydroxy-7a tétrahydro-5,6,7,7a 4H-furo[3,2-c] pyridine-one-2, chlorhydrate :

A une solution de 21 g (0,048 mole) de produit obtenu dans l'étape précédente, on ajoute 50 $cm^3$ d'une solution aqueuse d'acide chlorhydrique 3N et maintient le mélange à sa température de reflux pendant une heure; un précipité apparaît dans le milieu. On refroidit à température ambiante, et isole le précipité; il est alors lavé à l'eau froide puis à l'éthanol et séché; on recueille 12g de poudre blanche qui fonde en se décomposant vers 220-230° C (rendement : 78%).

c) (chloro-2 benzyl)-5 tétrahydro-5,6,7,7a 4H-furo[3,2-c] pyridine-one-2 :

A 10 g (0,0316 mole) de chlorhydrate de (chloro-2 benzyl)-5 hydroxy-7a tétrahydro-5,6,7,7a 4H-furo[3,2-c] pyridine-one-2 dans 350 $cm^3$ de soude 0,1N, on ajoute par portions 0,6 g (0,0158 mole) de borohydrure de sodium; on laisse le milieu sous agitation à température ambiante pendant 3 heures. Puis on ajoute 100 $cm^3$ d'acide chlorhydrique 12N. Après une heure à température ambiante, le pH du milieu est porté à environ 8 par addition de bicarbonate de sodium en poudre; on extrait alors à l'éther éthylique; la phase organique est lavée à l'eau, puis séchée sur sulfate de sodium et on évapore à sec.

On recueille une résine incolore que l'on concrétise dans l'éther isopropylique.

On recueille 7,5g de poudre blanche, F = 74° C (rendement : 90%).

d) le t-butyrate de (chloro-2 benzyl)-5 tétrahydro-4,5,6,7 furo[3,2-c] pyridyle-2 est préparé à partir du produit obtenu à l'étape précédente, en appliquant le mode opératoire de l'exemple 2 avec le tertiobutylate de potassium et le chlorure de pivaloyle. Le chlorhydrate fond à 160° C.

RMN$^1$H(80MHz,DMSOd6); 1,35(s,9H); 3,00-3,20(m,2H); 3,55-3,90(m,2H); 4,10(s,1H); 4,70(s,1H); 6,05(s,1H); 7,50-7,80(m,3H); 8,10-8,30(m,1H).

EXEMPLE 19

Composé de formule I dans laquelle Z = S; m = 1; n = 2; $R_2$ = $COOCH_3$; R′ = 2-$ClC_6H_4$; $R_1$ = $CH_3$ (référence SR 26571).

Ce composé est obtenu avec 98% de rendement par action de l'acétate d'isopropényle sur l'α-[oxo-2 hexahydro-2,4,5,6,7,7a thiéno[3,2-c] pyridyl-5] (chloro-2 phényl)-acétate de méthyle en appliquant le mode opératoire décrit à l'exemple 1.

Amine RMN$^1$H (250 MHz,$CDCl_3$) : 2,23(s,3H); 2,73-2,77 (m,2H); 2,85-2,89(m,2H); 3,51(d,1H); 3,64(d,3H); 3,69(s,1H); 4,89(s,1H); 5,27(s,1H); 7,22-7,41(m,3H); 7,64-7,68(m,1H).

Le chlorhydrate fond à 96° C.

Le produit de départ est préparé avec 58% de rendement en appliquant la méthode décrite à l'exemple 13 a), en faisant réagir 11,65 g d'α-chloro(chloro-2 phényl) acétate de méthyle au lieu du bromo-1 éthyl benzène; son chlorhydrate fond à 130° C.

EXCEMPLE 20

Composé de formule I dans laquelle Z = S; m = 1; n = 2; $R_2$ = $COOCH_3$; R′ = 2-$ClC_6H_4$; $R_1$ = C-$(CH_3)_3$ (référence SR 26859) :

On dissout 1,3 g (0,0047 mole) du chlorhydrate de t-butyrate de tétrahydro-4,5,6,7 thiéno[3,2,c] pyridyle-2 obtenu à l'exemple 16 dans 30 ml de diméthylformamide avec 0,94 g (0,0094 mole) de $KHCO_3$ et 1,24 g (0,047 mole) de bromo-2 (chloro-2 phényl) acétate de méthyle. Le milieu est maintenu sous agitation à 60° C pendant 2 heures, puis versé dans un mélange de 2 volumes d'eau et 2 volumes d'acétate d'éthyle; la phase organique est décantée, séparée. L'huile restant après élimination du solvant est chromatographiée sur une colonne de silice en éluant avec un mélange toluène/acétate d'éthyle (9/1).

Le chlorhydrate du produit final, isolé avec 66% de rendement, fond à 103 ° C.
RMN¹H (250MHz,DMSOd6) : 1,39(s,9H); 3,05(m,2H); 3,43 (m,2H); 3,84 (s,3H); 4,12(s,2H); 5,54(s,1H); 6,66-(s,1H); 7,71-7,75(m,3H); 7,93-7,95(m,1H).

EXEMPLE 21

Composé de formule I dans laquelle Z = S; m = 1; n = 2; $R_2$ = $COOCH_3$; R′ = 2-$ClC_6H_4$; $R_1$ = CH-$(CH_3)_2$ (référence SR 26886).

Préparé par action de chlorure d'isobutyryle sur le composé (SR 26861), avec 80% de rendement, en appliquant le mode opératoire décrit à l'exemple 20. Le chlorhydrate de ce composé fond à 111 ° C.
RMN¹H(250 MHz,DMSOd6) : 1,77(d,6H); 2,83(h,1H); 2,99 (m,2H); 3,42(m,2H); 3,72(s,3H); 4,11(s,2H); 5,58-(s,1H); 6,56(s,1H); 7,48-7,65(m,3H); 7,90-7,94(m,1H).

EXEMPLE 22

Composé de formule I dans laquelle Z = S; m = 1; n = 2;

$R_2$ = CON 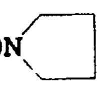 ;

R′ = 2-$ClC_6H_4$; $R_1$ = $CH_3$ (référence SR 26913).

Ce composé est préparé avec 43% de rendement en faisant réagir, selon le mode opératoire décrit à l'exemple 1, l'acétate d'isopropényle sur la N-[α-(oxo-2 hexahydro-2,4,5,6,7,7a thiéno[3,2-c] pyridyl-5) (chloro-2 phényl)]-acétyl pyrrolidine, obtenue en appliquant la méthode décrite dans l'exemple 11, du brevet FR-A-2 576 901.

Le chlorhydrate préparé dans l'éther éthylique fond à 135 ° C.
RMN¹H (80 MHz,DMSOd6) : 1,72-2,14(m,4H); 2,45(s,3H); 2,71-4,74(m,10H); 5,96(s,1H); 6,77(s,1H); 7,41-8,17 (m,4H).

EXEMPLE 23

Composé de formule I dans laquelle Z = S; m = 2; n = 1; $R_2$ = H; R′ = 2-$ClC_6H_4$; $R_1$ = $CH(CH_3)_2$ - (référence SR 26862).

a) (chloro-2 benzyl)-6 tétrahydro-5,6,7,7a 4H-thiéno[2,3-c] pyridine-one-2 :

A une solution refroidie à 0 ° C de 74 g (0,280 mole) de (chloro-2 benzyl)-6 tétrahydro-4,5,6,7 thiéno[2,3-c] pyridine préparé comme dans J. Heterocyclic Chem., 13 , 1347 (1976), dans 740 $cm^3$ de tétrahydrofuranne, on additionne goutte à goutte 210,4 $cm^3$ (0,337 mole) d'une solution de butyllithium 1,6 M dans l'hexane. A la fin de l'addition, on laisse revenir à température ambiante et agite pendant 15 minutes. Le milieu réactionnel est ensuite refroidi à -20 ° C et on ajoute goutte à goutte 90,8 $cm^3$ (0,337 mole) de tributylborate, dans 100 $cm^3$ de tétrahydrofuranne. L'introduction terminée, on agite pendant 1 heure à 10 ° C. La température est ensuite abaissée à -40 ° C et on ajoute, goutte à goutte, 79 $cm^3$ (0,700 mole) d'eau oxygénée à 30%. Un précipité intense se forme en cours d'addition. On laisse revenir le milieu réactionnel à température ambiante; après agitation pendant une nuit, le milieu est devenu homogène. On introduit alors 2 volumes d'eau et on extrait le produit final dans le dichlorométhane. La phase organique est lavée à l'eau et séchée. L'évaporation du solvant laisse un résidu que l'on purifie par filtration sur lit de silice (élution au dichlorométhane). Le chlorhydrate du produit final fond à 110 ° C (Rendement : 93%).
RMN¹H (80 MHz,DMSOd6) : 2,93-4,28(m,6H); 4,56(s,2H); 5,07-5,33(m,1H); 6,47(s,1H); 7,44-7,66(m,3H); 7,96-8,18(m,1H).

b) à 0 ° C, on ajoute, goutte à goutte 12,7 ml (0,020 mole) d'une solution 1,6M de butyllithium dans l'hexane, dans une solution de 5,15 g (0,019 mole) de (chloro-2 benzyl)-6 tétrahydro-5,6,7,7a 4H-thiéno [2,3-c] pyridine-one-2 dans 50 ml de tétrahydrofuranne. Après 15 minutes d'agitation toujours à 0 °C, on introduit goutte à goutte 2,1 ml (0,020 mole) de chlorure d'isobutyryle, puis laisse le milieu sous agitation pendant 1 heure à la température ambiante. On introduit alors 2 volumes d'eau et on extrait le produit final dans l'acétate d'éthyle; l'huile isolée après lavage à l'eau de la phase organique et évaporation du solvant est chromatographiée sur une colonne de silice, en éluant avec un mélange toluène/acétate d'éthyle (9/1-v/v). Le chlorhydrate du produit final fond à 111 °C. Rendement 40%.

RMN$^1$H(250 MHz,DMSOd6) : 1,20(d,6H); 2,80-2,90(m,1H); 2,90-3,15(m,1H); 3,45-3,70(m,3H); 4,35(s,2H); 4,59(s,2H); 6,62(s,1H); 7,40-7,65(m,3H); 8,05-8,15(m,1H).

EXEMPLE 24

Composé de formule I dans laquelle Z = S; m = 2; n = 1; $R_2$ = H; R' = 2-$ClC_6H_4$; $R_1$ = $C(CH_3)_3$ - (référence SR 26808).

A 0 °C, on ajoute goutte à goutte 7,74 ml (0,0124 mole) d'une solution 1,6M de butyllithium dans l'hexane, dans une solution de 3,3g (0,0118 mole) de (chloro-2 benzyl)-6 tétrahydro-5,6,7,7a 4H-thiéno [2,3-c] pyridine-one-2 dans 35 ml de tétrahydrofuranne. Après 15 minutes d'agitation toujours à 0 °C, on introduit goutte à goutte 1,48 ml (0,0120 mole) de chlorure de pivaloyle, puis laisse le milieu sous agitation pendant une nuit à la température ambiante. On introduit alors 2 volumes d'eau et on extrait le produit final dans l'acétate d'éthyle; l'huile isolée après lavage à l'eau de la phase organique et évaporation du solvant est chromatographiée sur une colonne de silice, en éluant avec un mélange toluène/acétate d'éthyle (9/1-v/v). Le chlorhydrate du produit final préparé dans l'éther éthylique, isolé avec 29% de rendement, fond à 145 °C.

RMN$^1$H(250 MHz,DMSOd6) : 1,27(s,9H); 2,87-2,99(m,2H); 3,39-3,57(m,2H); 4,33(s,2H); 4,58(s,2H); 6,63-(s,1H); 7,43-7,60(m,3H); 8,05-8,08(m,1H).

EXEMPLE 25

N-oxyde du composé de formule I dans laquelle Z = S; m =1; n = 2; $R_2$ = H; R' = 2-$ClC_6H_4$; $R_1$ = $CH_3$ (chlorhydrate; référence SR 26621).

On dissout 10g (0,031 mole) d'acétate de (chloro-2 benzyl)-5 tétrahydro-4,5,6,7 thiéno[3,2-c] pyridyl-2 obtenu selon l'exemple 1 dans 30 ml de chloroforme et on introduit dans la solution, maintenue à 0 °C, par portions, 6,3g (0,031 mole) d'acide méta-chloroperbenzoïque à 85%. Après la fin de l'addition, on laisse le milieu sous agitation à température ambiante pendant 2 heures, avant d'introduire au moins 0,04 mole de HCl sous forme d'une solution 2N dans l'éther éthylique. Le précipité formé, constitué du chlorhydrate du N-oxyde, est isolé par filtration. F = 112 °C - Rendement 79%.

RMN$^1$H (80 MHz,DMSOd6) : 2,45(s,3H); 3,10-3.45(m,2H); 4,02-4,50(m,2H); 4,72-5,11(m,2H); 5.33(s,2H); 6,72 (s,1H); 7,42-7,90(m,3H); 7,95-8,21(m,1H).

EXEMPLES 26 à 39

Les N-oxydes des composés de formule I suivants ont été préparés en appliquant le mode opératoire de l'exemple 25 aux amines hétérocycliques de formule I obtenues selon les procédés décrits dans les exemples précédents.

| Ex. | SR | Z | m | n | $R_2$ | R' | $R_1$ | F (chlorhydrate) | rendement en sel |
|---|---|---|---|---|---|---|---|---|---|
| 26 | 26832 | S | 1 | 2 | H | $2\text{-}ClC_6H_4$ | $C_3H_7$ | 128°C | 86 % |
| 27 | 26833 | S | 1 | 2 | H | $2\text{-}ClC_6H_4$ | $CH(CH_3)_2$ | 140°C | 67 % |
| 28 | 26831 | S | 1 | 2 | H | $2\text{-}ClC_6H_4$ | $C(CH_3)_3$ | 158°C | 87 % |
| 29 | 26725 | S | 1 | 2 | $COOCH$ | $2\text{-}ClC_6H_4$ | $CH_3$ | 100°C | 62 % |
| 30 | 26914 | S | 1 | 2 | CON 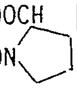 | $2\text{-}ClC_6H_4$ | $CH_3$ | 144°C | 63 % |
| 31 | 26986 | S | 1 | 2 | H | $C_6H_5$ | $C(CH_3)_3$ | 175°C | 82 % |
| 32 | 27354 | S | 1 | 2 | $CH_3$ | $C_6H_5$ | $C(CH_3)_3$ | 105°C | 71 % |
| 33 | 26977 | S | 1 | 2 | H | $4\text{-}ClC_6H_4$ | $C(CH_3)_3$ | 160°C | 83 % |
| 34 | 27262 | S | 1 | 2 | H | $4\text{-}OCH_3C_6H_4$ | $C(CH_3)_3$ | 165°C | 81 % |
| 35 | 27165 | S | 1 | 2 | H | $CH_3$ | $C(CH_3)_3$ | 186°C | 98 % |
| 36 | 27325 | S | 1 | 2 | H | $C_3H_7$ | $C(CH_3)_3$ | 116°C | 79 % |
| 37 | 26916 | S | 1 | 2 | H | $2\text{-}ClC_6H_4$ | $C_6H_5$ | 170°C | 88 % |
| 38 | 26998 | S | 2 | 1 | H | $2\text{-}ClC_6H_4$ | $C(CH_3)_3$ | 118°C | 48 % |
| 39 | 26993 | O | 1 | 2 | H | $2\text{-}ClC_6H_4$ | $C(CH_3)_3$ | 160°C | 90 % |

Spectres RMN$^1$H des chlorhydrates de ces produits :

| Ex. | δ(ppm) : |
|---|---|
| 26 | (250 MHz,CDCl3) : 1,00(t,3H) ; 1,74(q,3H) ; 2,52(t,2H) ; 3,02-3,12(m,2H) ; 3,38-3,51(m,1H) ; 3,93-4,04(m,1H) ; 4,48-4,59(m,2H) ; 4,79(d,1H) ; 5,31(d,1H) ; 5,42(d,1H) ; 6,34(s,1H) ; 7,39-7,50 (m,3H) ; 8,18-8,21(m,1H). |
| 27 | (250 MHz,CDCl3) : 1,29(d,6H) ; 2,79(h,1H) ; 3,05-3,14(m,1H) ; 3,43-3,49(m,1H) ; 3,91-4,02 (m,1H) ; 4,51-4,57(m,2H) ; 4,81(d,1H) ; 5,32(d,1H); 5,43(d,1H) ; 6,36(s,1H) ; 7,42-7,51(m,3H) : 8,21-8,24(m,1H). |
| 28 | (250 MHz,CDCl3) : 1,30(s,9H) ; 3,03-3,10(m,1H) ; 3,39-3,51(m,1H) ; 3,99-4,01(m,1H) ; 4,48-4,61 (m,2H) ; 4,81(d,1H) ; 5,32(d,1H) ; 5,42(d,1H) ; 6,35(s,1H) ; 7,39-7,50(m,3H) : 8,17-8,21(m,1H). |
| 29 | (80 MHz,CDCl3) : 2,35(s,3H) ; 2,79-3,31(m,2H) ; 3,95(s,3H) ; 4,20-5,55(m,4H) ; 6,55(s,1H) ; 6,98(s,1H) ; 7,54-7,87(m,3H) ; 8,29-8,56(m,1H). |
| 30 | (80 MHz,DMSOd6) : 1,55-1,93(m,4H) ; 2,26(s,3H) ; 3,48-5,32(m,10H) ; 6,33 et 6,39(2s,1H) ; 6,55 et 6,60(2s,1H) ; 7,25-7,96(m,4H). |
| 31 | (80 MHz,DMSOd6) : 1,37(s,9H) ; 3,14-3,36(m,2H) ; 4,07-4,26(m,2H) ; 4,51(d,1H) ; 4,98(d,1H) ; 5,18(s,2H) ; 6,74(s,1H) ; 7,59-7,87(m,5H). |
| 32 | (250 MHz,DMSOd6) : 1,25(s,9H) ; 1,80-1,90(m,3H) ; 3,00-3,15(m,1H) ; 3,15-3,35(m,2H) ; 4,20-4,40(m,1H) 4,55-4,80(m,1H) ; 4,85-4,95(d,1H) ; 5,15-5,4 (m,1H) ; 6,55(d,1H) ; 7,40-7,70(m,4H) ; 7,85(s,1H). |
| 33 | (250 MHz,CDCl3) ; 1,32(s,9H) ; 3,00(m,1H) ; 3,31 (m,1H) ; 4,06-4,39(m,4H) ; 4,51(d,1H) ; 4,86(d,1H); 5,18(d,1H) ; 5,29(d,1H) ; 6,30(s,1H) ; 7,35(d,2H) ; 7,67(d,2H). |

| | |
|---|---|
| 34 | (250 MHz,DMSO) : 1,24(s,9H) ; 3,10(m,2H) ; 3,78(s,3H) ; 4,03(m,2H) ; 4,41(d,1H) ; 4,87(s,1H) ; 5,08(s,2H) ; 6,64(s,1H) ; 7,07(d,2H) ; 7,62(d,2H). |
| 35 | (250 MHz,DMSO) : 1,27(s,9H) ; 3,11(m,1H) ; 3,33(m,1H) ; 3,66(s,3H) ; 3,99(m,1H) ; 4,12(m,1H) ; 4,82(s,2H) ; 6,63(s,1H). |
| 36 | (250 MHz,DMSOd6) : 0,95(t,3H) ; 1,25(s,9H) ; 1,80-2,95(m,2H) ; 3,00-3,30(m,2H) ; 3,75-3,85 (m,2H) ; 3,85-3,97(m,1H) ; 4,10-4,20(m,1H) ; 4,75(s,2H) ; 6,60(s,1H). |
| 37 | (250 MHz,DMSOd6) : 3,12-3,32(m,2H) ; 4,05-4,40 (m,2H) ; 4,77(d,1H) ; 5,05(d,1H) ; 5,30(s,2H) ; 6,87(s,1H) ; 7,50-8,35(m,9H). |
| 38 | (80 MHz,DMSOd6) : 1,35(s,9H) ; 3,30-3,50(m,2H) ; 4,10-4,35(m,2H) ; 5,00(s,1H) ; 5,10(s,1H) ; 5,35(s,2H) ; 6,80(s,1H) ; 7,60-7,75(m,3H) ; 8,00-8,10(m,1H) ; 13,00(s,1H). |
| 39 | (80 MHz,DMSOd6) : 1,35(s,9H) ; 3,05-3,30(m,2H) ; 4,20-4,40(m,2H) ; 4,80(s,1H) ; 5,00(s,1H) ; 5,35(s,2H) ; 6,10(s,1H) ; 7,60-7,80(m,3H) ; 8,10-8,25(m,1H). |

EXEMPLE 40

Composé de formule I dans laquelle Z = S; m = 1; n = 2; $R_2$ = H; R′ = 2-$ClC_6H_4$; $R_1$ = $C_2H_5O$ (référence SR 26893).

On introduit à 0°C, goutte à goutte, 4,15 ml d'une solution 1,6M de butyllithium dans l'hexane dans une solution de 1,78 g (0,063 mole) de (chloro-2 benzyl)-5 tétrahydro-5,6,7,7a 4H-thiéno[3,2-c] pyridine-one-2 dans 30 ml de tétrahydrofuranne. Après 15 minutes d'agitation, toujours à 0°C, on ajoute 0,67 ml (0,00695 mole) de chloroformiate d'éthyle, puis laisse le milieu pendant plusieurs heures, sous agitation, à température ambiante. Après addition de 2 volumes d'eau, on extrait le produit final avec le dichloro méthane et le purifie, après évaporation du solvant, par chromatographie sur une colonne de silice en éluant avec un mélange toluène/acétate d'éthyle (9/1-v/v). On obtient ainsi 1,4 g du produit recherché sous forme d'une huile jaune. L'oxalate, sel formé d'une molécule d'acide oxalique et d'une molécule d'amine de formule I, préparé dans l'éthanol, fond à 202°C.

Amine de formule I : RMN$^1$H : (80 MHz,$CDCl_3$) : 1,37(t,3H); 2,79-2,85(m,4H); 3,52(s,2H); 3,80(s,2H); 4,31-(q,2H); 6,33(s,1H); 7,18-7,38(m,3H); 7,51-7,55(m,1H).

EXEMPLE 41

Composé de formule I dans laquelle Z = S; m = 1; n = 2; $R_2$ = H; R′ = 2-$ClC_6H_4$; $R_1$ = $(CH_3)_2CH$-$CH_2$-O (référence SR 26896).

Préparé avec 49% de rendement en appliquant le mode opératoire de l'exemple 40, ce composé donne un oxalate qui fond à 168°C.

Amine de formule I : RMN$^1$H : (250 MHz,DMSOd6) : 0,92(d,6H); 1,96(m,1H); 2,79-2,82(m,2H); 2,97-3,01-(m,2H); 3,62(s,2H); 3,95(s,2H); 4,03(d,2H); 6,55(s,1H); 7,34-7,49(m,3H); 7,56-7,60(m,1H).

EXEMPLE 42

Composé de formule I dans laquelle Z = S; m = 1; n = 2; $R_2$ = H; R' = 2-$ClC_6H_4$; $R_1$ = $(CH_3)_3CO$ (référence SR 26887).

On introduit 33,2 ml d'une solution aqueuse de NaOH 1N dans une solution de 5g (0,0158 mole) de (chloro-2 benzyl)-5 tétrahydro-5,6,7,7a 4H-thiéno [3,2-c] pyridine-one-2 dans 50 ml de dioxanne, puis 5,17 g (0,0237 mole) de carbonate de ditertiobutyle. Après quelques heures d'agitation à température ambiante, on ajoute 2 volumes d'eau et 2 volumes d'acétate d'éthyle; la phase organique est décantée, lavée et séchée et l'huile isolée après élimination du solvant, est purifiée par chromatographie sur une colonne de silice en éluant avec un mélange toluène/acétate d'éthyle (9/1-v/v).

On obtient ainsi 4,6g du produit recherché sous forme d'une huile jaune; l'oxalate, qui cristallise dans l'acétone fond à 166° C.

Amine de formule I : RMN$^1$H : (80 MHz,$CDCl_3$) : 1,56(s,9H); 2,83(m,4H); 3,53(s,2H); 3,81(s,2H); 6,32(s,1H); 7,22-7,34(m,4H).

EXEMPLE 43

Composé de formule I dans laquelle Z = S; m = 1; n = 2; $R_2$ = H; R' = 2-$ClC_6H_4$; $R_1$ = $C_6H_5CH_2O$ (référence SR 26888).

On introduit, goutte à goutte à 5° C, 50 ml d'une solution, dans le tétrahydrofuranne, de 5g (0,0179 mole) de (chloro-2 benzyl)-5 tétrahydro-5,6,7,7a 4H-thiéno[3,2-c] pyridine-one-2 dans 20 ml d'une solution de 2,11 g (0,0188 mole) de tertiobutylate de potassium dans le tétrahydrofuranne. Après 15 minutes d'agitation, on introduit dans le milieu, toujours à cette température, 3,2g (0,0188 mole) de chloroformiate de benzyle. Après 2 heures d'agitation à température ambiante, on ajoute 2 volumes d'eau et 2 volumes d'acétate d'éthyle. Après agitation, la phase organique est séparée, lavée à l'eau, séchée sur $Na_2SO_4$ anhydre et le solvant éliminé. L'huile résiduelle est purifiée par chromatographie sur colonne de silice avec l'acétate d'éthyle comme éluant pour donner avec 98% de rendement le produit final sous forme d'une huile. L'oxalate du produit recherché fond à 171° C.

Amine de formule I : RMN$^1$H (250 MHz,DMSOd6) : 2,79(m,2H); 2,95(m,2H); 3, 60(s,2H); 3,93(s,2H); 5,29-(s,2H); 6,56(s,1H); 7,33-7,49(m,8H); 7,55-7,59(m,1H).

EXEMPLE 44

Composé de formule I dans laquelle Z = S; m = 2; n = 1; $R_2$ = H; R' = 2-$ClC_6H_4$; $R_1$ = $(CH_3)_2CH$-$CH_2$-O (référence SR 26809).

Ce composé a été préparé en appliquant le mode opératoire décrit dans l'exemple 40. L'oxalate du produit cherché, cristallisé dans l'acétone, fond à 168° C. Rendement 48%.

RMN$^1$H (250 MHz,DMSOd6) : 0,92(d,6H); 1,96(m,1H); 2,79-2,82(m,2H); 2,97-3,01(m,2H); 3,62(s,2H); 3,95-(s,2H); 4,03(d,2H); 6,55 (s,1H); 7,34-7,49(m,3H); 7,56-7,60(m,1H).

EXEMPLES 45 à 48

Les N-oxydes des composés des exemples 40 à 44 ont été préparés en appliquant le mode opératoire décrit à l'exemple 24 aux amines correspondantes. Les caractéristiques des produits obtenus figurent dans le tableau suivant :

| Ex. | SR | Z | m | n | $R_2$ | R' | $R_1$ | F (chlorhydrate) | rendement en sel |
|---|---|---|---|---|---|---|---|---|---|
| 45 | 26894 | S | 1 | 2 | H | 2-$ClC_6H_4$ | $C_2H_5$-O | 130° C | 94 % |
| 46 | 26898 | S | 1 | 2 | H | 2-$ClC_6H_4$ | $(CH_3)_2CHCH_2$-O | 140° C | 56 % |
| 47 | 26891 | S | 1 | 2 | H | 2-$ClC_6H_4$ | $C_6H_5$-$CH_2$-O | 113° C | 66 % |
| 48 | 26889 | S | 1 | 2 | H | 2-$ClC_6H_4$ | $(CH_3)_3C$-O | 126° C | 20 % |

Les caractéristiques des spectres RMN¹H des chlorhydrates de ces N-oxydes sont les suivants :

| Ex. | δ(ppm) : |
|---|---|
| 45 | (250 MHz,CDCl3) : 1,38(t,3H) ; 3,00-3,20(m,1H) ; 3,42-3,62(m,1H) ; 3,98-4,12(m,1H) ; 4,35(q,2H) ; 4,51-4,57(m,2H) ; 4,74(d,1H) ; 5,31(d,1H) ; 5,46(d,1H) ; 6,36(s,1H) ; 7,41-7,50(m,3H) ; 8,19-8,23(m,1H). |
| 46 | (250 MHz,CDCl3) : 0,97(d,6H) ; 1,99-2,05(m,1H) ; 2,93-3,13(m,1H) ; 3,34-3,51(m,1H) ; 4,02-4,05 (m,3H) ; 4,51-4,61(m,2H) ; 4,74(d,1H) ; 5,31(d,1H); 5,43(d,1H) ; 6,35(s,1H) ; 7,39-7,48(m,3H) : 8,15-8,19(m,1H). |
| 47 | (250 MHz,DMSOd6) : 3,07-3,25(m,2H) ; 4,03-4,31 (m,2H) ; 4,66-4,92(m,2H) ; 5,20(s,2H) ; 5,30(s,2H); 6,71(s,1H) ; 7,39-7,66(m,8H) ; 7,94-7,97(m,1H). |
| 48 | (250 MHz,DMSOd6) : 1,49(s,9H) ; 2,93-3,67(m,2H) ; 3,95-4,42(m,2H) ; 4,57-5,02(m,2H) ; 5,24(s,2H) ; 6,66(s,1H) ; 7,54-7,62(m,3H) ; 8,05-8,25(m,1H). |

EXEMPLE 49

Méthylsulfate du méthyl-5 ammonium du composé de formule I dans laquelle Z = S; m = 1; n = 2; $R_2$ = H; R' = 2-$ClC_6H_4$; $R_1$ = $(CH_3)_3C$ (référence SR 27168).

On dissout 8,35 g (0,0229 mole) de t-butyrate de (chloro-2 benzyl)-5 tétrahydro-4,5,6,7 thiéno[3,2-c] pyridyle-2 dans 85 ml d'acétone avec 2,89 g (0,0229 mole) de sulfate de diméthyle; après 12 heures d'agitation à température ambiante, on élimine les produits volatils sous pression réduite, le résidu est chromatographié sur une colonne de silice en éluant avec un mélange dichlorométhane/méthanol (9/1-v/v). Le méthylsulfate fond à 60° C; rendement 43 %.

On donnera ci-après des résultats des essais pharmacologiques mettant en évidence les propriétés des composés selon l'invention .

a) Inhibition in vitro de l'activité enzymatique des élastases.

On a étudié, selon une technique classique, la modification de la vitesse d'hydrolyse d'un substrat, connu comme spécifique des élastases, en présence et en absence du produit à étudier. Les essais ont été

effectués avec l'élastase pancréatique de porc et l'élastase leucocytaire humaine, extraite de crachats purulents; le substrat de l'enzyme était un tétrapeptide chromogène, le N-succinyl-L-alanyl-L-alanyl-L-propyl-L-leucyl-p-nitroanilide, noté Suc-Ala-Ala-Pro-LeupNA, qui par hydrolyse libère la p-nitroaniline dont l'apparition dans le milieu est suivie au spectrophotomètre; l'alpha-1 antitrypsine a été utilisée comme inhibiteur de référence. Les essais ont été effectués à 25 °C, la mesure spectrophotométrique étant faite à 410 nm.

La solution de substrat a été préparée en dissolvant 5,8 mg d'anilide dans 20 ml de solution tampon, contenant 24,22 g/l de Tris-HCl avec 754 mg/l de $CaCl_2$ ajustée à pH 8,8.

La solution d'enzyme, dans le même tampon, était à 0,1 g/l, tandis que les solutions de produit à tester dans le même tampon étaient de concentrations comprises entre 0,1 mM et 0,01 µM.

On a mélangé dans des tubes 200 µl de solution de substrat, 75 µl de solution tampon et 200 µl de solution du produit à tester, ou de solution tampon dans le cas des témoins. Après 20 minutes à 25 °C, on a placé le tube dans le spectrophomètre enregistreur et on a ajouté dans ce milieu 25 µl de la solution d'e zyme; la variation de la densité optique Δ DO a été enregistrée pendant 30 secondes.

Le pourcentage d'inhibition est défini comme étant la différence entre la variation de DO pour l'échantillon témoin et celle pour l'échantillon à tester divisée par la variation pour le témoin, multiplié par 100.

$$\% I = \frac{\Delta DO(\text{témoin}) - \Delta DO(\text{essai})}{\Delta DO(\text{témoin})} \times 100$$

Dans les tableaux I et II sont mentionnés les concentrations dans la solution finale du produit à tester pour lesquelles le pourcentage d'inhibition est de 50% ($CI_{50}$).

TABLEAU I

| Enzyme : élastase pancréatique de porc | | | | | |
|---|---|---|---|---|---|
| Composé à tester (SR) | $CI_{50}$ (µM) | Composé à tester (SR) | $CI_{50}$ (µM) | Composé à tester (SR) | $CI_{50}$ (µM) |
| α1-Antitrypsine | 1,2 | 26842 | 10 | 26986 | 2,2 |
| PCR 4094 | 2 | 26861 | 6,4 | 27354 | 10 |
| 26772 | 5,8 | 26938 | 3,6 | 26977 | 2,5 |
| 26769 | 7 | 26886 | 2,9 | 27262 | 1 |
| 26767 | 4,8 | 26913 | 32 | 27165 | 10 |
| 26829 | 8 | 26862 | 4 | 27325 | 15 |
| 26827 | 9 | 26832 | 7 | 26998 | 2,2 |
| 27261 | 4,5 | 26833 | 6 | 26993 | 1,7 |
| 27319 | 4,5 | 26831 | 5 | 26894 | 24 |
| 26984 | 3,6 | 26914 | 25 | 27168 | 1,6 |
| 27318 | 10 | | | | |

TABLEAU II

| Enzyme : élastase leucocytaire humaine | | | | | |
|---|---|---|---|---|---|
| Composé testé (SR) | $CI_{50}$ (μM) | Composé testé (SR) | $CI_{50}$ (μM) | Composé testé (SR) | $CI_{50}$ (μM) |
| α1-Antitrypsine | 1,2 | 27318 | 3 | 27354 | 2,6 |
| PCR 4094 | 3,5 | 26842 | 3,2 | 26977 | 1,8 |
| 26772 | 2,6 | 26861 | 2,6 | 27262 | 2,6 |
| 26769 | 30 | 26938 | 3,1 | 27165 | 1,2 |
| 26767 | 3,4 | 26886 | 30 | 27325 | 0,7 |
| 26829 | 24 | 26862 | 50 | 26998 | 2,2 |
| 26827 | 3,4 | 26832 | 5,6 | 26993 | 3,1 |
| 27261 | 3,2 | 26833 | 3,6 | 26887 | 50 |
| 27319 | 3,4 | 26831 | 2,5 | 27889 | 50 |
| 26984 | 2,6 | 26986 | 2,3 | | |

b) Activité antiagrégante plaquettaire chez le rat.

Selon une technique décrite notamment dans FR-A-2 567 901, on a étudié l'inhibition de l'agrégation plaquettaire dans du sang prélevé sur des animaux traités, lorsque l'on y ajoute de l'ADP ou du collagène; les produits à étudier étaient administrés par voie orale. Les SR 26766 et SR 26827 ont une activité particulièrement remarquable, puisqu'à la dose de 100 mg/kg, ils donnent un pourcentage d'inhibition de l'agrégation, respectivement, de 61 % et 48% pour l'ADP et de 62 % et 21 % pour le collagène.

## Revendications

1. Composés de formule

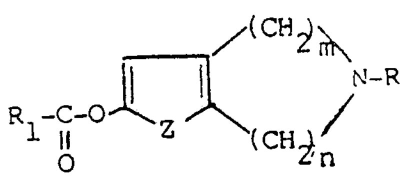

I

dans laquelle $R_1$ est choisi parmi les groupes $R_3$ et $OR_3$ dans lesquels $R_3$ est choisi parmi les groupes alkyle en $C_1$ à $C_6$, phényle et benzyle;

R est choisi parmi H et les groupes $CHR_2R'$ dans lesquels $R_2$ est choisi parmi H, les groupes alkyle en $C_1$ à $C_4$, les groupes $-COOR_4$, $R_4$ étant choisi parmi H, un groupe alkyle en $C_1$ à $C_4$ et benzyle, les groupes $-CONR_5R_6$, $R_5$ et $R_6$ indépendamment l'un de l'autre étant choisis parmi H, les groupes alkyle en $C_1$ à $C_4$ et benzyle ou $R_5$ et $R_6$ forment avec l'azote auquel ils sont rattachés un hétérocyle saturé en $C_4$-$C_8$; $R'$ est choisi parmi H, les groupes alkyle en $C_1$ à $C_4$ et phényle substitués ou non par un ou des substituants choisis parmi les atomes d'halogène, les groupes alkyle en $C_1$ à $C_4$, alkoxy en $C_1$ à $C_4$, trifluorométhyle et nitro;

Z représente S ou O; m et n, identiques ou différents, valent 1 ou 2,

ainsi que leurs sels avec des acides pharmaceutiquement acceptables, les N-oxydes des composés de formule I et leurs sels avec des acides pharmaceutiquement acceptables; et les ammonium quaternaires résultant de la condensation des composés de formule I avec un halogénure ou un sulfate d'alkyle en $C_1$ à $C_4$.

2. Composés selon la revendication 1, caractérisés en ce que $R_1$ représente un alkyle en $C_1$ à $C_6$.

3. Composés selon la revendication 1 ou 2, caractérisés en ce que $R'$ représente un groupe phényle éventuellement substitué par un substituant choisi parmi les atomes d'halogène, les groupes alkyle en $C_1$ à

$C_4$, alkoxy en $C_1$ à $C_4$, trifluorométhyle et nitro, et $R_2$ est choisi parmi $COOR_4$ et $CONR_5R_6$.

4. Composés selon la revendication 1 ou 2, caractérisés en ce que R′ représente un groupe phényle éventuellement substitué par un substituant choisi parmi les atomes d'halogène, les groupes alkyle en $C_1$ à $C_4$, alkoxy en $C_1$ à $C_4$, trifluorométhyle et nitro, et $R_2$ est choisi parmi H et les groupes alkyle en $C_1$ à $C_4$.

5. Composés selon l'une des revendications 1 à 4, caractérisés en ce que m vaut 1 et n vaut 2.

6. Composés selon l'une des revendications 1 à 4, caractérisés en ce que m vaut 2 et n vaut 1.

7. Composés selon la revendication 1, caractérisés en ce que Z est S, m vaut 1 ou 2, $R_1$ est un alkyle, $R_2$ est H et R′ représente un groupe phényle, substitué ou non et ses sels pharmaceutiquement acceptables.

8. Procédé de préparation des composés de formule I de l'une des revendications 1 à 7, caractérisé en ce que :

a) on fait réagir sur l'énolate du composé de formule II

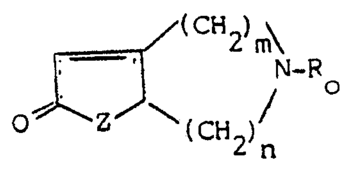

dans laquelle Z, m, n, ont la même signification que dans la revendication 1 et $R_o$ est un groupe protecteur labile en milieu acide, un dérivé réactif de $R_1COOH$ choisi parmi les chlorures et anhydrides d'acides ou le chloroformiate et le carbonate correspondants pour obtenir un composé de formule II a)

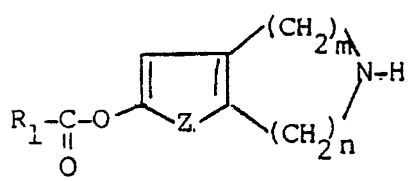

dans laquelle $R_1$ a la même signification que dans la revendication 1,

b) on élimine le groupe $R_o$ par action d'un acide anhydre, et lorsque R′ est différent de H

c) on alkyle l'amine secondaire par action de Y-$CHR_2R'$ avec Y choisi parmi Cl, Br et $R_7SO_3$, $R_7$ étant un alkyle en $C_1$ à $C_4$ en un groupe phényle.

9. Procédé de préparation des composés de formule I de l'une des revendications 1, 2 et 4 à 7, dans lesquels $R_2$ est choisi parmi H et les groupes alkyle en $C_1$ à $C_4$, caractérisé en ce que l'on fait réagir sur l'énolate du composé de formule

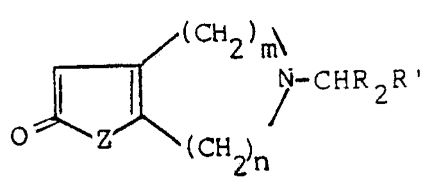

dans laquelle Z, m, n et R′ ont les mêmes significations que dans la formule I, un dérivé réactif de $R_1COOH$, choisi parmi les chlorures et anhydrides d'acides, le chloroformiate ou le carbonate correspondants.

10. Procédé de préparation des N-oxydes des composés de formule I de la revendication 1, caractérisé en ce que la fonction amine du composé obtenu selon l'une des revendications 8 ou 9 est oxydée par réaction avec un acide peroxycarboxylique.

11. Procédé de préparation des sels de N-oxydes selon la revendication 10, caractérisé en ce que le sel est obtenu par addition d'un acide fort dans le milieu en fin d'oxydation.

12. Composition pharmaceutique comprenant au moins l'un des composés selon l'une des revendications 1 à 7.

Revendications pour les Etats contractants suivants: ES, GR

1. Procédé de préparation des composés de formule

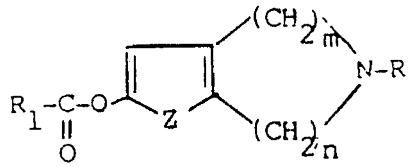

I

dans laquelle $R_1$ est choisi parmi les groupes $R_3$ et $OR_3$ dans lesquels $R_3$ est choisi parmi les groupes alkyle en $C_1$ à $C_6$, phényle et benzyle;

R est choisi parmi H et les groupes $CHR_2R'$ dans lesquels $R_2$ est choisi parmi H, les groupes alkyle en $C_1$ à $C_4$, les groupes $-COOR_4$, $R_4$ étant choisi parmi H, un groupe alkyle en $C_1$ à $C_4$ et benzyle, les groupes $-CONR_5R_6$, $R_5$ et $R_6$ indépendamment l'un de l'autre étant choisis parmi H, les groupes alkyle en $C_1$ à $C_4$ et benzyle ou $R_5$ et $R_6$ forment avec l'azote auquel ils sont rattachés un hétérocyle saturé en $C_4$-$C_8$; $R'$ est choisi parmi H, les groupes alkyle en $C_1$ à $C_4$ et phényle substitués ou non par un ou des substituants choisis parmi les atomes d'halogène, les groupes alkyle en $C_1$ à $C_4$, alkoxy en $C_1$ à $C_4$, trifluorométhyle et nitro;

Z représente S ou O; m et n, identiques ou différents, valent 1 ou 2,

ainsi que leurs sels avec des acides pharmaceutiquement acceptables, les N-oxydes des composés de formule I et leurs sels avec des acides pharmaceutiquement acceptables; et les ammonium quaternaires résultant de la condensation des composés de formule I avec un halogénure ou un sulfate d'alkyle en $C_1$ à $C_4$, caractérisé en ce que :

a) on fait réagir sur l'énolate du composé de formule II

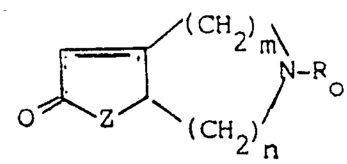

dans laquelle Z, m, n, ont la même signification que dans la revendication 1 et $R_o$ est un groupe protecteur labile en milieu acide, un dérivé réactif de $R_1COOH$ choisi parmi les chlorures et anhydrides d'acides ou le chloroformiate et le carbonate correspondants pour obtenir un composé de formule II a)

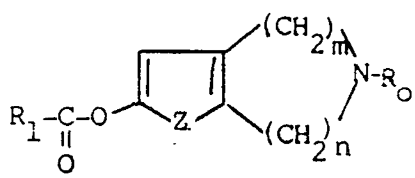

dans laquelle $R_1$ a la même signification que dans la formule I,

b) on élimine le groupe $R_o$ par action d'un acide anhydre, et lorsque R est différent de H

c) on alkyle l'amine secondaire par action de $Y-CHR_2R'$ avec Y choisi parmi Cl, Br et $R_7SO_3$, $R_7$ étant un alkyle en $C_1$ à $C_4$ en un groupe phényle.

2. Procédé selon la revendication 1, caractérisé en ce que l'on prépare des composés de formule I dans laquelle R représente un alkyle en $C_1$ à $C_6$.

3. Procédé selon la revendication 1 ou 2, caractérisé en ce que l'on prépare des composés de formule I dans laquelle $R'$ représente un groupe phényle éventuellement substitué par un substituant choisi parmi les atomes d'halogène, les groupes alkyle en $C_1$ à $C_4$, alkoxy en $C_1$ à $C_4$, trifluorométhyle et nitro, et $R_2$ est choisi parmi $COOR_4$ et $CONR_5R_6$.

4. Procédé selon la revendication 1 ou 2, caractérisé en ce que l'on prépare des composés de formule I dans laquelle $R'$ représente un groupe phényle éventuellement substitué par un substituant choisi parmi les atomes d'halogène, les groupes alkyle en $C_1$ à $C_4$, alkoxy en $C_1$ à $C_4$, trifluorométhyle et nitro, et $R_2$ est choisi parmi H et les groupes alkyle en $C_1$ à $C_4$.

5. Procédé selon l'une des revendications 1 à 4, caractérisé en ce que l'on prépare des composés de

formule I dans laquelle m vaut 1 et n vaut 2.

6. Procédé selon l'une des revendications 1 à 4, caractérisé en ce que l'on prépare des composés de formule I dans laquelle m vaut 2 et n vaut 1.

7. Procédé selon la revendication 1, caractérisé en ce que l'on prépare des composés de formule I dans laquelle Z est S, m vaut 1 ou 2, $R_1$ est un alkyle, $R_2$ est H et $R'$ représente un groupe phényle, substitué ou non et ses sels pharmaceutiquement acceptables.

8. Procédé de préparation selon l'une des revendications 1, 2 et 4 à 7 dans laquelle $R_2$ est choisi parmi H et les groupes alkyle en $C_1$ à $C_4$, caractérisé en ce que l'on fait réagir sur l'énolate du composé de formule

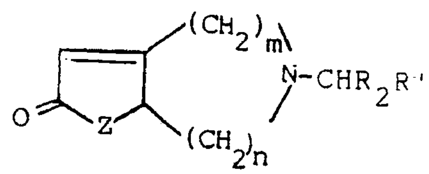

dans laquelle Z, m, n et $R'$ ont les mêmes significations que dans la formule I, un dérivé réactif de $R_1COOH$, choisi parmi les chlorures et anhydrides d'acide, le chloroformiate ou le carbonate correspondants.

9. Procédé de préparation des N-oxydes des composés de formule I de la revendication 1, caractérisé en ce que la fonction amine du composé obtenu est oxydée par réaction avec un acide peroxycarboxylique.

10. Procédé de préparation des sels de N-oxydes selon la revendication 9, caractérisé en ce que le sel est obtenu par addition d'un acide fort dans le milieu en fin d'oxydation.

11. Procédé de préparation d'une composition pharmaceutique comprenant au moins l'un des composés préparé selon l'une des revendications 1 à 10, caractérisé en ce que l'on associe un composé de formule I, son N-oxyde, ses sels avec un acide minéral ou organique pharmaceutiquement acceptable ou ses ammoniums quaternaires aux excipients ou véhicules pharmaceutiquement acceptables.

Office européen des brevets

# RAPPORT DE RECHERCHE EUROPEENNE

Numero de la demande

EP 90 40 2711

## DOCUMENTS CONSIDERES COMME PERTINENTS

| Catégorie | Citation du document avec indication, en cas de besoin, des parties pertinentes | Revendication concernée | CLASSEMENT DE LA DEMANDE (Int. Cl.5) |
|---|---|---|---|
| A | EP-A-0 058 341 (Dr. KARL THOMAE)<br>* Revendication 1 *<br>--- | 1 | C 07 D 491/04<br>C 07 D 495/04<br>A 61 K 31/40<br>A 61 K 31/435<br>A 61 K 31/55 //<br>(C 07 D 491/04<br>C 07 D 307:00<br>C 07 D 209:00 )<br>(C 07 D 491/04<br>C 07 D 307:00<br>C 07 D 221:00 )<br>(C 07 D 491/04<br>C 07 D 307:00<br>C 07 D 223:00 )<br>(C 07 D 495/04<br>C 07 D 333:00<br>C 07 D 209:00 )<br>-/- |
| D,A | EP-A-0 054 442 (SANOFI)<br>* Revendication 1 *<br>--- | 1 | |
| D,A | EP-A-0 192 535 (SANOFI)<br>* Revendication 1 *<br>----- | 1 | |
| | | | **DOMAINES TECHNIQUES RECHERCHES (Int. Cl.5)** |
| | | | C 07 D 491/00<br>C 07 D 495/00<br>A 61 K 31/00 |

Le présent rapport a été établi pour toutes les revendications

| Lieu de la recherche | Date d'achèvement de la recherche | Examinateur |
|---|---|---|
| LA HAYE | 09-01-1991 | VOYIAZOGLOU D. |

**CATEGORIE DES DOCUMENTS CITES**

X : particulièrement pertinent à lui seul
Y : particulièrement pertinent en combinaison avec un autre document de la même catégorie
A : arrière-plan technologique
O : divulgation non-écrite
P : document intercalaire

T : théorie ou principe à la base de l'invention
E : document de brevet antérieur, mais publié à la date de dépôt ou après cette date
D : cité dans la demande
L : cité pour d'autres raisons

& : membre de la même famille, document correspondant

EPO FORM 1503 03.82 (P0402)